# EUROPEAN PATENT SPECIFICATION

(11) **EP 3 280 796 B1**
(45) Date of publication and mention of the grant of the patent: **11.09.2019**
(21) Application number: 16725903.5
(22) Date of filing: 07.04.2016
(51) Int. Cl.: C12N 5/0783, G01N 33/50, A61P 37/00, A61P 43/00

(54) **POPULATION OF HUMAN LYMPHOID PRECURSORS, METHOD FOR THEIR IDENTIFICATION AND USES THEREOF**
POPULATION VON MENSCHLICHEN LYMPHOIDVORLÄUFERN, VERFAHREN ZU DEREN IDENTIFIZIERUNG UND VERWENDUNGEN DAVON
POPULATION DE PRÉCURSEURS DE LYMPHOÏDES HUMAINS, LEUR PROCÉDÉ D'IDENTIFICATION ET LEURS UTILISATIONS

(30) Priority: 09.04.2015 IT UB20150079
(43) Date of publication of application: 14.02.2018
(73) Proprietor: Universita' degli Studi di Genova, 16126 Genova (IT)
(72) Inventor: DE MARIA, Andrea, I-16124 Genova (IT); MORETTA, Lorenzo, I-16145 Genova (IT); BOZZANO, Federica, I-16149 Genova (IT); MARRAS, Francesco, I-16166 Genova (IT)
(74) Representative: Calogero, Ida
(86) International application number: PCT/IB2016/051984
(87) International publication number: WO 2016/162827

(56) References cited:
- WO-A2-2014/028453
- US-A1- 2009 123 442
- SANCHEZ-CORREA B ET AL: "Decreased expression of DNAM-1 on NK cells from acute myeloid leukemia patients", IMMUNOLOGY AND CELL BIOLOGY, vol. 90, no. 1, 8 March 2011 (2011-03-08), pages 109-115, XP055128288, ISSN: 0818-9641, DOI: 10.1038/icb.2011.15
- MOUSTAKI A ET AL: "Effect of the simultaneous administration of glucocorticoids and IL-15 on human NK cell phenotype, proliferation and function", CANCER IMMUNOLOGY IMMUNOTHERAPY, vol. 60, no. 12, 26 June 2011 (2011-06-26) , pages 1683-1695, XP019980941, ISSN: 1432-0851, DOI: 10.1007/S00262-011-1067-6
- BOZZANO F ET AL: "'Emergency exit' of bone-marrow-resident CD34+DNAM-1brightCXCR4+-committed lymphoid precursors during chronic infection and inflammation", NATURE COMMUNICATIONS, vol. 6, 8109, 5 October 2015 (2015-10-05), XP055236468, ISSN: 2041-1723, DOI: 10.1038/ncomms9109 -& "Supplementary Information", Nature Communications, 5 October 2015 (2015-10-05), XP055236469, DOI: 10.1038/ncomms9109 Retrieved from the Internet: URL:http://www.nature.com/ncomms/2015/1510 05/ncomms9109/extref/ncomms9109-s1.pdf [retrieved on 2015-12-15]

## Description

The present invention relates to the identification of a new cell population of human lymphoid precursors characterized by the expression of markers such as CD34, DNAM-1 (CD226) and CXCR4, the relative method for their identification and the relative uses in the therapeutic and diagnostic field.

Natural cell killers (NK) belong to the innate immunity compartment and play an important role in the defense against viruses, bacteria, cancer cells. In human beings they represent about 5-10% of the lymphocytes circulating in the peripheral blood and are divided into two cell subsets: CD56^{dim} and CD56^{bright}.

NK cells originate from CD34+ hematopoietic stem cells, their maturation begins at the level of the bone marrow and is completed through different development stages, on the periphery (Caligiuri M.A., 2008; Moretta L., 2010).

Chronic infections from viruses such as HIV, HCV and tuberculosis can alter the phenotype and function of the NK cells (De Maria A. 2003; Bozzano F., 2011; Marras F., 2013). All of these conditions differ from each other in the phenotype and distribution of the NK cells, but they have a persistent modulation/activation of the same NK cells, in common. The wide involvement of NK cells suggests the possibility of an increased turnover and regeneration on the part of CD34+ cells. An increased turnover with an exhaustion of CD34+ cells was previously observed in patients suffering from HIV with a continuous viral replication (Sauce D., 2011). In the field of immunology, the identification of chronic inflammation markers (such as, for example, in chronic inflammatory or infectious diseases or in autoimmune diseases) plays an important role at both a therapeutic and diagnostic level.

Inflammation markers such as CRP (C-reactive Protein), ESR (erythrocyte sedimentation rate), fibrinogen, α1 and α2 globulins evaluated in serum electrophoresis, C3 and C4 produced by the liver, are normally used in clinics for monitoring the progression of the disease or effect of the therapy. The use of these common markers, however, does not allow the identification of fine differences in the control of diseases to allow an evaluation of the treatment ad hoc (as in the case of diseases such as HIV or chronic obstructive pulmonary disease (COPD)).

The authors of the present invention have now identified a cell population of human lymphoid precursors characterized by the expression of markers such as CD34, DNAM-1 (CD226) expressed in bright mode and CXCR4 (hereunder identified as cell population of human lymphoid precursors CD34⁺ DNAM^{BRIGHT} CXCR4⁺) whose presence significantly correlates with inflammatory processes of a chronic nature.

Said cell population, which has never been described to date, has been observed in the peripheral blood of patients infected with HIV, tuberculosis (TB) and hepatitis C (HCV) but also in non-infectious diseases with an important inflammatory component, such as chronic obstructive pulmonary disease (COPD) and auto-inflammatory diseases such as the PAPA syndrome. Furthermore, as mentioned above, this population has been observed in the peripheral blood of patients chronically infected by HIV, and hepatitis C, whereas in healthy subjects, it is present at the level of the bone marrow and cannot be identified in the blood of the umbilical cord (UCMC), a classical source of CD34+ cells. The different localization of the cell population, object of the present invention, makes its use as a diagnostic marker of the chronic inflammatory state of a human being, of unquestionable benefit.

When cultivated *in vitro,* the cell population CD34⁺ DNAM^{BRIGHT} CXCR4⁺ gives rise to NK cells in an advanced state of maturation and lymphocytes T CD4+, CD8+, alpha beta (αβ), gamma delta (γδ). DNAM^{BRIGHT} CXCR4⁺ cells can differ from NK cells in the presence of a culture medium consisting of the medium Myelocult H5100 (StemCell Technologies, Vancouver, British Columbia, Canada), human serum AB (ICN Pharmaceuticals Italy, Milano, Italy), human recombinant cytokines, such as FLT3-L, IL7, SCF, IL-15 (PeproThec, London, UK) (see Marras F., 2012). It is evident from literature that the T-cell differentiation from C34+ stem cells *in vitro* envisages culture with a medium in which cytokines SCF, IL-3, IL-7 are present and mononuclear cells irradiated as feeder (see Sanchez M., 1998).

In literature, there is only one work that analyzes a cell population identified as CD34+ DNAM+ (Ma D., 2005) which, however, when cultivated *in vitro* under suitable culture conditions such as the presence of 100 ng/mL of thrombopoietin (TPO), gives rise to megakaryocytes and not to NK cells and/or T cells CD4+, CD8+, alpha beta (αβ), gamma delta (γδ).

The particular plasticity of the cell population isolated, makes it capable of being the object of the development of specific therapies. The role it plays, in fact, could be of definite interest in the field of hematology (bone marrow transplant) due to the capacity of contemporaneously giving rise to mature NK cells and T cells and consequently their involvement in the control and/or development of GvHD (Graft versus Host Disease) or in the field of the control of responses with respect to viral infections, such as hepatitis C (HCV) and HIV.

Consequently, the percentage of cell population CD34⁺ DNAM^{BRIGHT} CXCR4⁺ circulating in the peripheral blood always correlates with the presence of chronic inflammation and in some cases selected with the degree of inflammation assessed, for example, through the dosage of CRP (C-reactive protein).

In the diseases analyzed of an infectious nature (such as, for example, HIV, hepatitis C, tuberculosis) and non-infectious nature (such as, for example, COPD and the PAPA syndrome) linked by a chronic inflammation condition, the presence of the cell population CD34⁺ DNAM^{BRIGHT} CXCR4⁺ in the peripheral blood of pathological subjects is observed with respect to healthy subjects in whom the localization is only observed at a bone marrow level as it is also absent in the cord blood (UCMC).

A possible explanation from a pathogenic point of view is the following. Chronic inflammation is associated with a bone remodelling also including the endosteal niches with the result that the retention mechanism of the hematopoietic stem cells in the bone marrow dependent on the cytokine axis MMP-9/CXCR4, is modified (Takayanagi H., 2007; Morbach H., 2013; Kollet O., 2006). The cell population CD34⁺ DNAM^{BRIGHT} CXCR4⁺ probably subjected to a migration deriving from the endosteal niches following chronic inflammation and bone remodelling can also represent a useful turnover/reconstitution indicator of the NK and T lymphoid cells and levels of chronic inflammation.

Therefore it is an object of the present invention a cell population of human lymphoid precursors isolated from peripheral blood of a human being suffering from a state of chronic inflammation or autoimmunity or from the bone marrow of a healthy human being, characterized by the expression on the cell membrane of the surface markers CD34, DNAM-1 (CD226) expressed in bright mode, and CXCR4. More specifically, the cell population of human lymphoid precursors according to the invention is further characterized by the expression or one or more of the following surface markers: CD69, CD38, HLA-DR, and by the absence of one or more of the following surface markers: CD3, CD14, CD19, CD16, CD56, CD117, CDW328 (P75/AIRM1), CD161, CD11C and CD123.

The human being suffering from a state of chronic inflammation can be an adult or pediatric individual. In particular, said state of chronic inflammation can be typical of chronic inflammatory diseases such as chronic obstructive pulmonary disease (COPD), rare auto-inflammatory diseases such as the PAPA syndrome, autoimmune diseases (such as, for example, systemic sclerosis, systemic lupus erythematosus, rheumatoid arthritis) or chronic infectious diseases such as, for example HIV, hepatitis C and tuberculosis.

A further aspect of the present disclosure relates to the cell population of human lymphoid precursors isolated as described above, for use in the medical field, for example as a therapeutic target.

Another object of the invention relates to the use of the cell population CD34⁺ DNAM^{BRIGHT} CXCR4⁺ identified above, as marker of the state of chronic inflammation in an adult or pediatric human individual. In particular, said state of chronic inflammation can be typical of chronic inflammatory diseases such as chronic obstructive pulmonary disease (COPD), rare auto-inflammatory diseases such as the PAPA syndrome, autoimmune diseases (such as, for example, systemic sclerosis, systemic lupus erythematosus, rheumatoid arthritis) or chronic infectious diseases such as, for example HIV, hepatitis C and tuberculosis.

A further aspect of the disclosure relates to a kit for the identification of the cell population CD34⁺ DNAM^{BRIGHT} CXCR4⁺, comprising the following components:
A) anti-DNAM-1 monoclonal antibodies (CD226) (i.e. group IgG1, clone F22);
B) mixture of anti-human CD3, anti-human CD14, anti-human CD19 monoclonal antibodies, conjugated with fluorochrome, preferably the same fluorochrome;
C) anti-human CD56 monoclonal antibodies conjugated with fluorochrome;
D) anti-human CD16 monoclonal antibodies conjugated with fluorochrome;
E) anti-human CD34 monoclonal antibodies conjugated with fluorochrome;
F) anti-human CXCR4 monoclonal antibodies (i.e. group IgG2a, BD).

The fluorochromes can be selected from PeCy7, APC, V500, BV510 and FITC.

According to said aspect of the present disclosure, the anti-CD56 monoclonal antibodies are conjugated with PeCy7 (Beckman Coulter); the anti-CD3, -anti-CD14, -anti-CD19 monoclonal antibodies are conjugated in APC or V500 or BV510 (BD Pharmingen); anti-CD16 monoclonal antibodies are conjugated with FITC (BD Pharmingen).

The above kit can additionally contain one or more sterile 5 ml polystyrene tubes.

In virtue of the expression of chemokine receptors and cytokines such as CXCR4, CX3CR1, CXCR1, the cells object of the invention, can themselves become the target for the development of pharmacological treatment as the control of their migration from the bone marrow could become important in the control of the pathological state of Graft versus Host Disease, a disease occurring in patients subjected to bone marrow transplants.

Furthermore, due to the capacity of producing T lymphocytes without thymic stimulation, any other manipulation (removal, induction, etc.) effected *in vivo* of the above population used as therapeutic target, can be used for modulating autoimmunity, giving origin to an advanced biological therapy in substitution of the classical immunosuppressive drug therapy.

A further object of the present invention therefore relates to the use of the cell population CD34⁺ DNAM^{BRIGHT} CXCR4⁺ as a source for creating NK cells or CD4+, CD8+, alpha beta (αβ) and gamma delta (γδ) T lymphocytes differentiated *in vitro,* that can be then re-infused into the patient in need of treatment.

The identification and selection of donors with a different percentage of this new population object of the invention, could lead to different results in transplants and consequently direct the choice of donor on the basis of the percentage of CD34⁺ DNAM^{BRIGHT} cells present in the donor.

A further object of the present invention therefore relates to a method for the identification of the cell population of human lymphoid precursors CD34⁺ DNAM^{BRIGHT} CXCR4⁺ from a sample of peripheral blood or bone marrow, comprising the following steps:
a) primary detection of the presence of the marker DNAM-1 (CD226) by means of anti-DNAM-1 (CD226) monoclonal antibodies and subsequent detection with secondary antibodies;
b) primary detection of the surface markers CD3, CD14, CD16, CD19, CD56 by means of anti-human CD3, anti-human CD14, anti-human CD16, anti-human CD19, anti-human CD56 monoclonal antibodies directly conjugated with fluorochrome;
c) cytofluorimetric analysis.

The cytofluorimetric analysis reveals the quality of the bright expression profile, when the fluorescence intensity reaches the fourth log decade.

The method can further include a primary detection of the surface marker CD34 and/or CXCR4 by means of anti-human CD34 monoclonal antibodies conjugated with fluorochrome (i.e. with PeCy7, BD) and/or anti-human CXCR4 monoclonal antibodies (i.e. group Ig2A, BD, non-conjugated).

The fluorochromes are preferably selected from PeCy7, APC, V500, BV510 and FITC.

In a preferred embodiment, antibodies conjugated with phycoerythrin (PE) for the secondary detection, are used.

Finally, an object of the present invention relates to the cell population of human lymphoid precursors that can be isolated through the identification method illustrated above (described in detail in Example 1).

The present invention is now described for illustrative but non-limiting purposes, according to a preferred embodiment with particular reference to the enclosed figures, in which:
- Figure 1a shows the results of the cytofluorimetric analysis of the expression of DNAM-1 (CD226) on peripheral blood mononuclear cells (PBMC) of a HIV patient and a healthy donor (HD);
- Figure 1b shows the results of the cytofluorimetric analysis of the presence of CD34⁺DNAM^{bright} cells present in the bone marrow of a healthy donor;
- Figure 2 shows the results of the cytofluorimetric analysis of the expression of DNAM-1 on peripheral blood mononuclear cells (PBMC) of a HCV- or TB-infected patient and healthy donor (HD), a patient suffering from COPD and the PAPA Syndrome;
- Figure 3 shows the results of the cytofluorimetric analysis of the expression of markers such as CXCR4, CD69, HLADR, CD38, CD117 on CD34⁺DNAM^{bright} cells of HIV patients compared with CD34⁺DNAM^{neg} cells (classical CD34);
- Figure 4 shows the graphs indicating the percentage of maturing T and NK cells obtained after culture of the CD34⁺DNAM-1^{bright} and CD34⁺DNAM-1^{neg} cells.

The invention is described with preferred examples in the following experimental section. Said examples should be considered as being purely illustrative and non-limiting of the same invention.

### EXAMPLE 1: Identification of the cell population of human lymphoid precursors CD34⁺ DNAM^{BRIGHT} CXCR4⁺ from peripheral blood

The example illustrates the method for identifying the cell population of human lymphoid precursors CD34⁺ DNAM^{BRIGHT} CXCR4⁺ according to the invention from peripheral blood mononuclear cells (PBMC).

### Sample collection

20 ml of peripheral blood were collected by venipuncture from a healthy donor and diluted with a further 20 ml of RPMI (Lonza/BioWhittaker).

### Separation of peripheral blood mononuclear cells (PBMC)

Two sterile 50 ml polystyrene tubes were prepared into which 10 ml of Ficoll-Hypaque were poured.

The peripheral blood was stratified and diluted to point 2 in the 50 ml tube containing Ficoll.

A centrifuge was effected at 2,000 rpm for 20 minutes at room temperature and without brakes to have a gradient centrifugation.

A culture medium consisting of 500 ml of RPMI was prepared, to which 10% of fetal calf serum was added, together with 2 mM L-glutamine, and a mixture of antibiotics penicillin and streptomycin 1%, which is then filtered through coffee filters with 0.22 µm pores (Millipore).

At the end of the centrifugation, the central ring between the Ficoll phases containing PBMCs was collected in a sterile 15 ml U-bottom tube with a 5 ml pipette. The volume was brought to 15 ml with RPMI and the whole mixture was centrifuged at 1100 rpm for 6 minutes at 4°C.

The supernatant was emptied and the pellet of cells was re-suspended in 15 ml of culture medium which was centrifuged at 1500 rpm for 5 minutes at 4°C.

The supernatant was emptied and the pellet of PBMC was brought to a volume of 10 ml with culture medium.

The cells were counted withdrawing 10 µl of PBMC and adding them to 90 µl of Tripan Blue (Sigma) previously diluted 1:5. 10 µl were withdrawn and put in the counting chamber.

The disposable sterile U-bottom polystyrene tubes were identified as follows:
tube A) CD56-CD3-CD14-CD19-CD16-CNTPE (negative control for the fluorochrome PE)
tube B) CD56-CD3-CD14-CD19-CD16-DNAM-1(CD226)

### Immunoreaction with monoclonal antibodies

200,000 PBMC cells were then distributed in the U-bottom tubes previously identified at a concentration of 1,000,000/ml. 50 µl of anti-DNAM-1 monoclonal antibodies (group IgG1, clone F22) were added at a concentration of 0.1 µg/ml.

Incubation was then effected for 20 minutes at 4°C.

2ml of PBS (Lonza) were added and a centrifuge was effected at 1500 rpm for 5 minutes at 4°C.

The supernatant was emptied and 50 µl of anti-murine IgG1 goat secondary monoclonal antibody conjugated with phycoerythrin (PE; BD Biosciences) diluted to the concentration indicated in PBS, were added.

Incubation was then effected for 20 minutes at 4°C. 2 ml of PBS (Lonza) were added and a centrifuge was effected at 1500 rpm for 5 minutes at 4°C.

2.5 µl of anti-CD56 monoclonal antibody conjugated with PeCy7 (Beckman Coulter) were added to each tube, and 2 µl of each of the anti-CD3, -anti-CD14, - anti-CD19 monoclonal antibodies conjugated in APC or V500 or BV510 (BD Pharmingen) and 2 µl anti-CD16 monoclonal antibody directly conjugated with FITC (BD Pharmingen).

Incubation was then effected for 20 minutes at 4°C. 2 ml of PBS (Lonza) were added and a centrifuge was effected at 1500 rpm for 5 minutes at 4°C.

The supernatant was emptied and 200 µl of PBS were added to each tube.

The tubes were acquired by means of a FACSCanto II cytofluorimeter (Becton Dickinson), and the PBMCs were identified by means of Forward and Side Scatter and 10,000 events were acquired in the gate CD56-CD3-CD14-CD19-CD16-.

The data acquired were analyzed with FlowJo (TreeStar) software.

### EXAMPLE 2: Study on the different localization of the cell population CD34⁺ DNAM^{BRIGHT} CXCR4⁺

The example illustrates the data relating to the presence of the population in the peripheral blood of subjects suffering from HIV, and the localization at the level of the bone marrow in healthy subjects (HD).

For this purpose, 60 HIV-infected non-viremic patients (i.e. with no detectable viremia in the plasma) were recruited, treated with antiretroviral therapy (cART) after obtaining informed consent from the patient in the study approved by the Ethics Committee of the San Martino-IST Hospital in Genoa, Italy. 15 healthy adult donors pertaining to the blood bank of the Gaslini Hospital in Genoa, Italy, were then recruited.

The procedure adopted for the sample collection, separation of the peripheral blood mononuclear cells (PBMC), immunofluorescence with monoclonal antibodies, is the same as that described in Example 1, applied to the withdrawal of peripheral blood from healthy subjects or subjects suffering from HIV.

The monoclonal antibodies used were PeCy5- anti-CD56 (Immunotech-Coulter, Marseille, Francia) and PeCy7-directly conjugated anti-CD34, allophycocyanin-conjugated (APC) anti-CD3, anti-CD14, anti-CD19, FITC conjugated anti-CD16 (BD Pharmigen, San Jose, CA, USA).

The samples thus prepared were acquired with a FACSCantoII (BD) cytofluorimeter and the data analyzed with FlowJO (TreeStar) software.

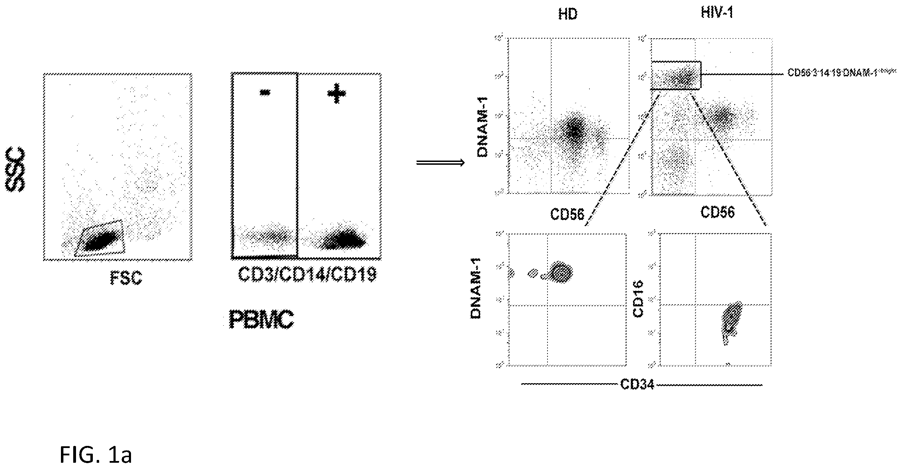

The results of the analysis carried out on peripheral blood mononuclear cells (PBMC) deriving from the patient suffering from HIV and the healthy donor (HD) are illustrated in Figure 1a.

In particular, Figure 1a shows how only in the peripheral blood mononuclear cells (PBMC) of HIV-infected patients, selected on the basis of the lack of expression of the markers CD3, CD14, CD19, is it possible to observe the cell population DNAM^{bright}CD56⁻ (upper line). Furthermore, the cells in question DNAM^{bright}CD56⁻ prove to co-express the marker CD34, whereas they do not express the receptor CD16 FcRIII (lower line). The population thus identified by the expression of the marker CD34⁺ and negative for the expression of the markers CD3, CD14, CD19, CD16, CD56, proves to be DNAM-1 (CD226) 78% positive (within a range of 41-98%). The absence of the markers CD3, CD14, CD19, CD16, CD56 is also indicated as Lin neg.

In order to stimulate the frequency and dimension of the population, the percentage present in samples coming from HIV-positive patients undergoing cART antiretroviral treatment was evaluated, comparing the data obtained with those deriving from the group of healthy donors (HD).

In the HD group, the population object of the study was present in a percentage lower than 1% in the PBMCs of 13/15 healthy donors and around 1-3% in 2/15 healthy donors.

On comparing the data with HIV-positive patients, the percentage in the latter proved to be extremely high: over 30% of the HIV-infected patients showed a percentage of the population higher than 5% which in selected cases reached 30-40% of the PBMCs selected on the basis of the lack of expression of the markers CD3, CD14, CD19 with a high statistical significance (**p<0.01 obtained with Mann-Witheny Test effected with JMP statistical software).

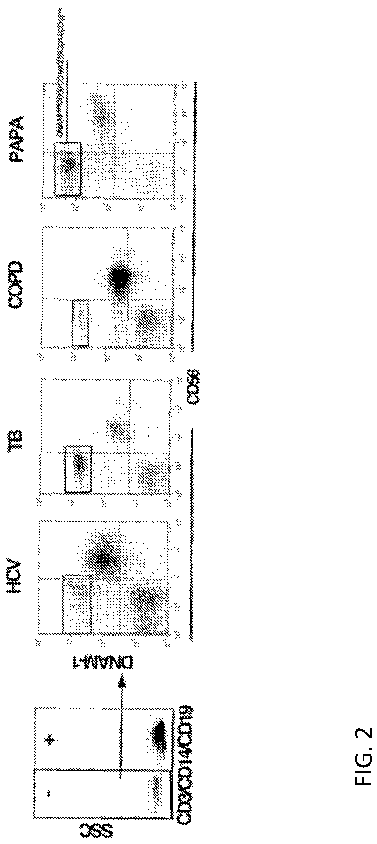

A similar situation was observed in patients suffering from hepatitis C, tuberculosis and in patients suffering from COPD and the PAPA syndrome (see Example 3, Figure 2).

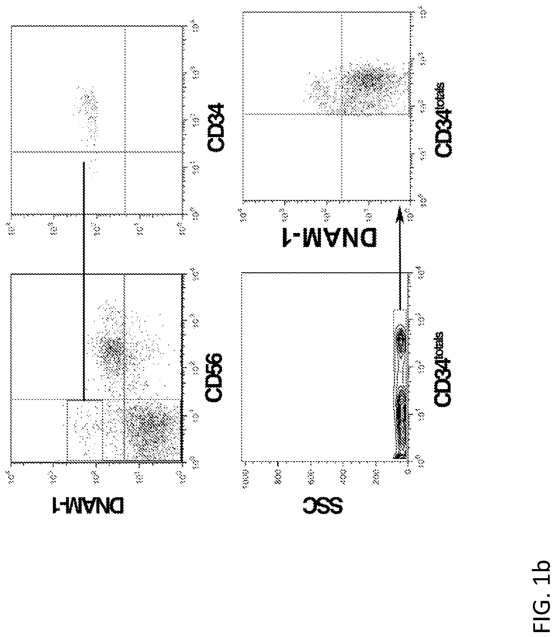

Figure 1b, on the other hand, shows the cytofluorimetric analysis of the presence of CD34⁺DNAM^{bright} cells present in the bone marrow of a healthy donor (HD).

For this purpose, after obtaining informed consent, 15 healthy adult donors pertaining to the blood bank of the Gaslini Hospital in Genoa, were recruited and 10 samples of bone marrow were collected at the Unit of Hematology and Bone Marrow Transplant Hospital of San Martino-IST Genoa, Italy.

The analysis was effected through staining with monoclonal antibodies conjugated with fluorochromes (direct or indirect immunofluorescence). The monoclonal antibodies used were PeCy5- anti-CD56 (Immunotech-Coulter, Marseille, France); and PeCy7-directly conjugated anti-CD34, allophycocyanin-conjugated (APC) anti-CD3, anti-CD14, anti-CD19, FITC conjugated anti-CD16 (BD Pharmigen, San Jose, CA, USA).

The samples thus prepared were acquired with a FACSCantoII (BD) cytofluorimeter and the data analyzed with FlowJO (TreeStar) software.

The cytofluorimetric analysis reveals the presence at the level of the bone marrow of the population object of the study (upper line) and that 10% of the population CD3-, CD14-, CD19-, CD56-, CD16-, CD34+ co-expresses DNAM-1 (CD226) (lower line).

The upper line of Figure 1b shows the expression of the marker DNAM-1 (CD226) on the cells CD3- CD14- CD19- CD34+ of the bone marrow. The lower line shows the expression of the marker DNAM-1 (CD226) on the cells CD34+ deriving from bone marrow.

In 6 samples of bone marrow from a healthy donor, the population CD34+DNAM-1^{bright} represented 15.6 ± 7.66% (average +/- DS) of the cell population of the bone marrow.

### EXAMPLE 3: Identification of the cell population CD34⁺ DNAM^{BRIGHT} CXCR4⁺ in patients suffering from hepatitis C, tuberculosis, the COPD syndrome and the PAPA syndrome.

For this purpose, after obtaining the informed consent of the patients, 27 HCV-infected patients, 27 patients suffering from post primary tuberculosis reactivation, 10 patients suffering from chronic obstructive pulmonary disease (COPD) were recruited at the Department of Internal Medicine of the Hospital of San Martino-IST in Genoa, Italy, and finally 3 patients suffering from the PAPA syndrome enrolled at the G Gaslini Institute in Genoa, Italy.

The same procedure was used as described in Example 1 for the sample collection, preparation of the tubes, immunofluorescence with monoclonal antibodies.

The analysis was effected through staining with monoclonal antibodies conjugated with fluorochromes (direct or indirect immunofluorescence). The monoclonal antibodies used were PeCy5- anti-CD56 (Immunotech-Coulter, Marseille, France); and PeCy7-directly conjugated anti-CD34, allophycocyanin-conjugated (APC) anti-CD3, anti-CD14, anti-CD19, FITC conjugated anti-CD16 (BD Pharmigen, San Jose, CA, USA).

The samples thus prepared were acquired with a FACSCantoII (BD) cytofluorimeter and the data analyzed with FlowJO (TreeStar) software.

The results are shown in Figure 2 in which a comparison is made of the cytofluorimetric analysis of the expression of DNAM-1 (CD226) on peripheral blood mononuclear cells (PBMC) of patients suffering from hepatitis C, tuberculosis, the COPD syndrome and PAPA Sydrome, respectively.

Among the PBMCs selected on the basis of the lack of expression of the markers CD3, CD14, CD19, it can be noted that the cell population DNAM^{bright}CD56⁻ can also be observed in other infectious diseases such as hepatitis C, tuberculosis, or in inflammatory-based diseases such as COPD or with an autoinflammatory component such as the PAPA syndrome.

### EXAMPLE 4: Analysis of the expression of the receptors CD69, HLADR, CD117 on the cell populations CD34⁺ DNAM^{BRIGHT} CXCR4⁺ and CD34⁺ DNAM^{neg} CXCR4^{neg} deriving from patients suffering from HIV.

For this purpose, 60 HIV-infected non-viremic patients (i.e. with no detectable viremia in the plasma), treated with antiretroviral therapy (cART), were recruited, after obtaining informed consent from the patient.

The same procedure was adopted as described in Example 1 for the sample collection and separation of the peripheral blood mononuclear cells (PBMC).

The antibodies used for revealing the expression of the markers CXCR4, CD69, HLADR, CD38 and CD117 are anti-CXCR4 monoclonal antibodies, anti-CD117 monoclonal antibodies and anti-CD38 monoclonal antibodies conjugated with PerCp/Cy5.5 (BD Pharmigen, San Jose, CA, USA), anti-CD69 monoclonal antibodies conjugated with fluorochrome PE (BioLegend, San Diego, CA, USA), HLADR (clone D1-12), IgG2a (kindly donated by Dr. R.S. Accolla of the Università di Insubria, Varese, Italy).

### Immunoreaction with monoclonal antibodies

200,000 PBMC cells were then distributed in the U-bottom tubes previously identified at a concentration of 1,000,000/ml.
- tube 1 CD56-CD3-CD14-CD19-CD16-CNTPE-CNTFITC (negative control for the fluorochrome PE E FITC)
- tube 2 CD56-CD3-CD14-CD19-CD16-DNAM-1(CD226)-CD34-CXCR4
- tube 3 CD56-CD3-CD14-CD19-CD16-DNAM-1(CD226)-CD34-CD69
- tube 4 CD56-CD3-CD14-CD19-CD16-DNAM-1(CD226)-CD34-HLADR
- tube 5 CD56-CD3-CD14-CD19-CD16-DNAM-1(CD226)-CD34-CD38
- tube 6 CD56-CD3-CD14-CD19-CD16-DNAM-1(CD226)-CD34-CD117

50 µl of anti-DNAM-1 (CD226) monoclonal antibodies were added at a concentration of 0.1 µg/ml in all the tubes and anti-CXCR4 at a concentration of 0.1 µg/ml in tube 2, anti-HLADR at a concentration of 0.1 µg/ml in tube 4. Incubation was then effected for 20 minutes at 4°C.

2ml of PBS (Lonza) were added in all the tubes and a centrifuge was effected at 1500 rpm for 5 minutes at 4°C.

The supernatant was emptied and 50 µl of anti-murine IgG1 goat secondary monoclonal antibody conjugated with phycoerythrin (PE; BD Biosciences) diluted to the concentration indicated in PBS, were added in all the tubes and 50 µl of anti-murine IgG2a goat secondary monoclonal antibody conjugated with FITC in tubes 2 and 4, diluted to the concentration indicated in PBS.

Incubation was then effected for 20 minutes at 4°C. 2 ml of PBS (Lonza) were added and a centrifuge was effected at 1500 rpm for 5 minutes at 4°C.

The supernatant was emptied and 2.5 µl of anti-CD56 monoclonal antibody conjugated with PeCy7 (Beckman Coulter) were added to each tube, and 2 µl of each of the anti-CD3, -anti-CD14, -anti-CD19 monoclonal antibodies conjugated in APC or V500 or BV510 (BD Pharmingen) and 2 µl anti-CD34 monoclonal antibody conjugated with PeCy7 (BD Pharmingen), whereas anti-CD 117 and anti-CD38 conjugated with PerCp/Cy5.5 (BD Pharmigen, San Jose, CA, USA), anti-CD69 conjugated with PE fluorochrome (BioLegend, San Diego, CA, USA), were added to tubes 3, 5, 6.

Incubation was then effected for 20 minutes at 4°C. 2 ml of PBS (Lonza) were added and a centrifuge was effected at 1500 rpm for 5 minutes at 4°C.

The supernatant was emptied and 200 µl of PBS were added to each tube.

The tubes were acquired by means of a FACSCanto II cytofluorimeter (Becton Dickinson), and the PBMCs were identified by means of Forward and Side Scatter and 10,000 events were acquired in the gate CD56-CD3-CD14-CD19-CD16-.

The data acquired were analyzed with FlowJo (TreeStar) software.

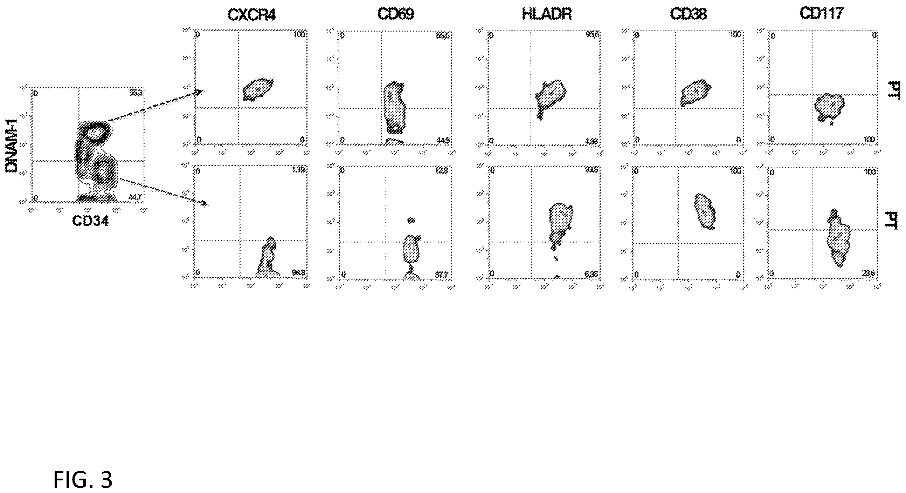

The results are indicated in Figure 3 in which a comparison is made of the cytofluorimetric analysis of the expression of markers such as CXCR4, CD69, HLADR, CD38, CD117 in the cell population CD34+ DNAM^{bright} CXCR4⁺ object of the present invention, in patients suffering from HIV, with respect to the same analysis carried out on CD34+DNAMneg (classical CD34) cells. The cell population object of the present invention shows the expression of CXCR4, CD69, HLADR, CD38, whereas it does not express CD117.

### EXAMPLE 5: Differentiation in vitro in NK and T cells starting from the cell population of human lymphoid precursors CD34⁺ DNAM^{BRIGHT} CXCR4⁺

For this purpose, 60 HIV-infected non-viremic patients (i.e. with no detectable viremia in the plasma), treated with antiretroviral therapy (cART), were recruited, after obtaining informed consent from the patient, and 10 samples of cord blood (UCMC) again after obtaining informed consent of the donor, collected at the Department of Gynecology of the Gaslini Hospital in Genoa, Italy.

The same procedure was adopted as described in Example 1 for the sample collection, separation of the peripheral blood mononuclear cells (PBMC), immunoreaction, and as described in Example 5 for the separation conditions and culture of the two CD34⁺ cell populations.

The monoclonal antibodies used for evaluating the markers on the different cell populations were: anti-CD4 (IgG1), anti-CD8 (IgG1), anti-TCRαβ (IgG1) (BD Pharmigen, San Jose, CA, USA), anti-NKG2D (BAT221, IgG1), anti-DNAM-1 (F22, IgG1) produced in the laboratory of the University of Genoa of Prof. A Moretta, Genoa, Italy; anti-CD56 conjugated with PeCy7 and anti-CD3 conjugated with FITC (BD Pharmigen, San Jose, CA, USA); PeCy5-anti-CD56 (Immunotech-Coulter, Marseille, France); and PeCy7-directly conjugated anti-CD34, allophycocyanin-conjugated (APC) anti-CD3, anti-CD14, anti-CD19, FITC conjugated anti-CD16 (BD Pharmigen, San Jose, CA, USA).

The samples prepared after immunoreaction were acquired with a FACSCantoII (BD) cytofluorimeter and the data analyzed with FlowJO (TreeStar) software.

### Separation conditions and culture of the CD34⁺ cells

The CD34⁺ cells were isolated from peripheral blood by means of immunomagnetic separation, using the CD34 MicroBead Kit (Miltenyi, Bergisch Gladbach, Germany).

The PBMC cells isolated as described in Example 1 were placed in a sterile 10 ml polystyrene tube, marked with anti-CD34 antibody in the quantities indicated in the above-mentioned kit, and incubated for 20 minutes in ice.

10 ml of buffer consisting of PBS, albumin were added in the concentrations indicated in the commercial kit and centrifuged at 1500 rpm for 5 minutes at 4°C. After discarding the supernatant and re-suspending the cells in the above buffer, they were passed through a column containing beads immersed in a magnetic field. The positive CD34 cells were thus selected, remaining anchored to the beads. They were subsequently detached from the column and collected following the instructions provided in the commercial kit.

The CD34 cells thus isolated were then marked with the anti-DNAM-1 monoclonal antibody at a concentration of 0.1 µg/ml and selected using the instrument cell sorter FACSAria (BD Biosciences) in order to obtain the highly purified populations of Lin-DNAM-1^{brignt} and Lin-DNAM-1-^{neg}.

The two populations selected were placed on 24-well plates at a concentration of 2-4x10^{4/}ml in a complete medium for precursors containing the medium Myelocult H5100 (StemCell Technologies, Vancouver, British Columbia, Canada) supplemented with human serum AB 10% (ICN Pharmaceuticals Italy, Milan, Italy), 5% fetal goat serum (FCS) and recombinant cytokines such as rhIL-15, rhIL-7,SCF, FLT3-L (PeproThec, London, UK) at a final concentration of 20 ng/mL.

An analysis of maturing NK cells starting from the plated precursors and T cells, was effected following the indications for direct and indirect immunofluorescence (see above).

The samples were then acquired by means of a FACSCanto II (Becton Dickinson) cytofluorimeter and the NK and T cells were identified by means of Forward and Side Scatter and 10,000 events were acquired.

The data acquired were analyzed with FlowJo (TreeStar) software.

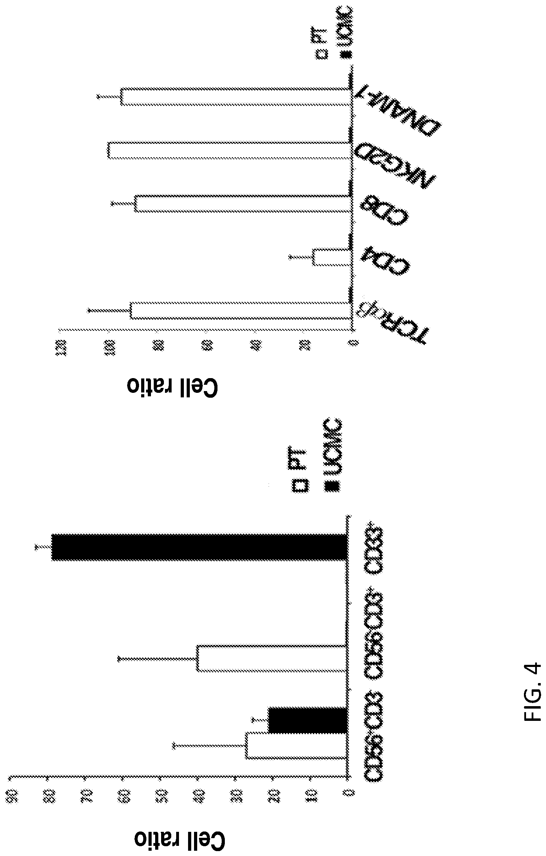

The results of the analysis of the percentage of maturing T and NK cells obtained after the culture of CD34⁺DNAM-1^{bright} and CD34⁺DNAM-1^{neg} cells are shown in Figure 4.

The histograms on the left represent the percentage of cells recovered after culture and revealed thanks to the direct and indirect immunofluorescence technique and acquisition of the sample by means of a cytofluorimeter.

A comparison can be observed between the cells, object of the patent, deriving from the patient and those deriving from the negative CD34+DNAM-1 (CD226) cells from cord blood of the healthy donor (UCMC).

The histograms on the right show the different expression percentage of the various receptors on the T cells (CD3+) deriving from the culture of CD34⁺DNAM-1^{bright} cells.

No lymphocyte T cells originating from the population CD34⁺DNAM^{neg} were observed and consequently no histogram could be constructed for the population CD34⁺DNAM^{neg} deriving from cord blood of the healthy donor (UCMC).

The statistical analysis was effected thanks to the use of JMP statistical software.

### BIBLIOGRAPHY

- Caligiuri MA. Blood 2008; 112:461-9.
- Moretta L. Blood 2010; 116:3689-91.
- De Maria A. et al. Eur. J. Immunol. 2003; 33 (9): 2410-8.
- Bozzano F. et al. Eur. J. Immunol. 2011;41 (10): 2905-14.
- Marras F. et al. Proc. Natl. Acad. Sci. USA. 2013; 110(29): 11970-5.
- Sauce D. et al. Blood 2011;117(19):5142-51.
- Ma D. et al. Eur. J. Haematol. 2005; 74(3):228-40.
- Marras F. et al. Eur. J. Immunol. 2012. 42: 2459-2470
- Sanchez M. et al. British Journal of Haematology, 1998, 103, 1198-1205
- Takayanagi H. Nat. Rev. Immunol. 2007; 7:292-304.
- Morbach H. Clinical Immunology 2013; 147:185-96.
- Kollet O., Nat Med 2006; 12:657-64.

## Claims

1. Human lymphoid precursors cell population isolated from peripheral human blood of a subject affected by a chronical inflammation or autoimmunity state or from the bone marrow of an healthy subject, **characterized by** the expression on the cell membrane of the surface markers CD34, DNAM-1 (CD226) expressed in bright mode, and CXCR4.

2. Human lymphoid precursors cell population according to claim 1, further **characterized by** the expression of at least one surface marker of the group consisting of CD69, CD38, HLA-DR, and/or by the absence of at least one of surface marker of the group consisting of CD3, CD14, CD19, CD16, CD56, CD117, CDW328 (P75/AIRM1), CD161, CD11C and CD123.

3. Use of the human lymphoid precursors cell population according to claim 1 or 2 as a marker of the chronic inflammation state in a human subject.

4. Use according to claim 3 of the population as a marker of chronic inflammation diseases, in particular the chronic obstructive pulmonary disease (COPD) or rare autoinflammatory diseases, in particular the PAPA syndrome.

5. Use according to claim 3 of the population as a marker of chronical infectious diseases selected among HIV, hepatitis C and tuberculosis.

6. Use according to claim 3 of the population as a marker of autoimmune diseases selected between systemic sclerosis, systemic lupus erythematosus and rheumatoid arthritis.

7. Use of the human lymphoid precursors cell population according to claim 1 or 2, as the source to originate *in vitro* differentiated Natural Killer cells and/or CD4+, CD8+, alpha beta (αβ) and gamma delta (γδ) T cells.

8. Method for the identification of the human lymphoid precursors cell population according to claim 1 or 2 in a peripheral blood or bone marrow sample, comprising the following steps:
a) primary detection of the presence of the marker DNAM-1 (CD226) by monoclonal antibodies anti-DNAM-1 and detection by secondary antibodies;
b) primary detection of the surface markers CD3, CD14, CD16, CD19, CD56 by monoclonal antibodies anti-CD3, anti-CD14, anti-CD16, anti-CD19, anti-CD56 conjugated with fluorochrome;
c) cytofluorimetric analysis.

9. Method according to claim 8, further comprising a detection step of the surface marker CD34 and/or CXCR4 by monoclonal antibodies.

10. Method according to claim 8 or 9, wherein said secondary antibodies of step a) are conjugated with phycoerythrin.

11. Method according to anyone of the claims 8-10, wherein the fluorochromes are selected among the group consisting of PeCy7, APC, V500, BV510 and FITC.

12. Human lymphoid precursors cell population isolable by the method according to anyone of the claims 8-11.

## Patentansprüche

1. Menschliche lymphoide Vorläuferzellpopulation, isoliert aus dem peripheren Blut von Personen, die von chronischen Entzündungen oder Autoimmunität betroffen sind, oder aus dem Knochenmark einer gesunden Person, **gekennzeichnet durch** die Expression in der Zellmembran der strahlenden Oberflächenmarker CD34, DNAM-1 (CD226) und CXCR4.

2. Menschliche lymphoide Vorläuferzellpopulation nach Anspruch 1, ferner **gekennzeichnet durch** die Expression von mindestens einem Oberflächenmarker aus der Gruppe bestehend aus CD69, CD38, HLA-DR, und/oder die Abwesenheit mindestens eines Oberflächenmarkers aus der Gruppe bestehend aus CD3, CD14, CD19, CD16, CD56, CD117, CDW328 (P75/AIRM1), CD161, CD11C und CD123.

3. Verwendung der menschlichen lymphoiden Vorläuferzellpopulation nach Anspruch 1 oder 2 als Marker des chronischen Entzündungsstatus in einer menschlichen Person.

4. Verwendung nach Anspruch 3 der Population als Marker von chronischen Entzündungskrankheiten, insbesondere der chronisch obstruktiven Lungenerkrankung (COPD) oder seltener autoentzündlicher Krankheiten, insbesondere des PAPA-Syndroms.

5. Verwendung nach Anspruch 3 der Population als Marker von chronischen Infektionskrankheiten, ausgewählt aus HIV, Hepatitis C und Tuberkulose.

6. Verwendung nach Anspruch 3 der Population als Marker von Autoimmunkrankheiten ausgewählt aus systemischer Sklerose, systemischem Lupus erythematodes und rheumatischer Arthritis.

7. Verwendung der menschlichen lymphoiden Vorläuferzellpopulation nach Anspruch 1 oder 2 als Quelle zur *In-Vitro*-Erzeugung differenzierter natürlicher Killerzellen und/oder CD4+, CD8+, Alpha-Beta-(αβ)- und Gamma-Delta-(γδ)-T-Zellen.

8. Verfahren zur Identifizierung der menschlichen lymphoiden Vorläuferzellpopulation nach Anspruch 1 oder 2 in einer Peripherblut- oder Knochenmarksprobe, umfassend folgende Schritte:
a) primäres Erfassen des Vorhandenseins des Markers DNAM-1 (CD226) durch monoklonale Antikörper Anti-DNAM-1 und Erfassen durch sekundäre Antikörper;
b) primäres Erfassen der Oberflächenmarker CD3, CD14, CD16, CD19, CD56 durch monoklonale Antikörper Anti-CD3, Anti-CD14, Anti-CD16, Anti-CD19, Anti-CD56 konjugiert mit Fluorochrom;
c) zytofluorimetrische Analyse.

9. Verfahren nach Anspruch 8, ferner umfassend einen Schritt des Erfassens des Oberflächenmarkers CD34 und/oder CXCR4 durch monoklonale Antikörper.

10. Verfahren nach Anspruch 8 oder 9, wobei die sekundären Antikörper aus Schritt a) mit Phycoerythrin konjugiert sind.

11. Verfahren nach einem der Ansprüche 8-10, wobei die Fluorochrome ausgewählt sind aus der Gruppe bestehend aus PeCy7, APC, V500, BV510 und FITC.

12. Menschliche lymphoide Vorläuferzellpopulation, isolierbar durch das Verfahren nach einem der Ansprüche 8-11.

## Revendications

1. Population cellulaire de précurseurs lymphoïdes humains, isolée du sang humain périphérique d'un sujet atteint d'inflammation chronique ou en état d'auto-immunité, ou de la moelle osseuse d'un sujet sain, **caractérisée par** l'expression sur la membrane cellulaire des marqueurs de surface CD34, DNAM-1 (CD226) exprimé en mode clair et CXCR4.

2. Population cellulaire de précurseurs lymphoïdes humains selon la revendication 1, **caractérisée en outre par** l'expression d'au moins un marqueur de surface du groupe constitué de CD69, CD38, HLA-DR et/ou par l'absence d'au moins un des marqueurs de surface du groupe composé de CD3, CD14, CD19, CD16, CD56, CD117, CDW328 (P75/AIRM1), CD161, CD11C et CD123.

3. Utilisation de la population cellulaire de précurseurs lymphoïdes humains selon la revendication 1 ou 2 comme marqueur de l'état d'inflammation chronique chez un sujet humain.

4. Utilisation de la population selon la revendication 3 comme marqueur de maladies inflammatoires chroniques, en particulier de la broncho-pneumopathie chronique obstructive (BPCO) ou de maladies auto-inflammatoires rares, en particulier du syndrome PAPA.

5. Utilisation de la population selon la revendication 3 comme marqueur de maladies infectieuses chroniques sélectionnées entre le VIH, l'hépatite C et la tuberculose.

6. Utilisation de la population selon la revendication 3 comme marqueur de maladies auto-immunes sélectionnées entre la sclérodermie systémique, le lupus érythémateux disséminé et la polyarthrite rhumatoïde.

7. Utilisation de la population cellulaire de précurseurs lymphoïdes humains selon la revendication 1 ou 2, comme source pour générer des cellules tueuses naturelles différenciées *in vitro* et/ou des cellules T CD4+, CD8+, alpha beta (αβ) et gamma delta (γδ).

8. Procédé d'identification de la population cellulaire de précurseurs lymphoïdes humains selon la revendication 1 ou 2 dans un échantillon de sang périphérique ou de moelle osseuse, comprenant les étapes suivantes :
a) la détection primaire de la présence du marqueur DNAM-1 (CD226) par des anticorps monoclonaux anti-DNAM-1 et la détection par des anticorps secondaires ;
b) la détection primaire des marqueurs de surface CD3, CD14, CD16, CD19 et CD56 par des anticorps monoclonaux anti-CD3, anti-CD14, anti-CD16, anti-CD19 et anti-CD56, conjugués au fluorochrome ;
c) l'analyse cytofluorimétrique.

9. Procédé selon la revendication 8, comprenant en outre une étape de détection du marqueur de surface CD34 et/ou CXCR4 par des anticorps monoclonaux.

10. Procédé selon la revendication 8 ou 9, dans lequel les anticorps secondaires en question à l'étape a) sont conjugués à la phycoérythrine.

11. Procédé selon l'une quelconque des revendications 8 à 10, dans lequel les fluorochromes sont choisis dans le groupe constitué de PeCy7, APC, V500, BV510 et FITC.

12. Population cellulaire de précurseurs lymphoïdes humains, isolable par le procédé selon l'une quelconque des revendications 8 à 11.
